(19) Europäisches Patentamt
European Patent Office
Office européen des brevets

(11) Veröffentlichungsnummer: **0 040 347**
**B1**

(12) # EUROPÄISCHE PATENTSCHRIFT

(45) Veröffentlichungstag der Patentschrift: **11.07.84**

(51) Int. Cl.³: **C 07 J 9/00**, C 07 J 13/00, C 07 J 41/00

(21) Anmeldenummer: **81103332.3**

(22) Anmeldetag: **02.05.81**

(54) **Neue Steroid-20-carbonsäureverbindungen und Verfahren zu ihrer Herstellung.**

(30) Priorität: **16.05.80 AT 2629/80**

(43) Veröffentlichungstag der Anmeldung: **25.11.81 Patentblatt 81/47**

(45) Bekanntmachung des Hinweises auf die Patenterteilung: **11.07.84 Patentblatt 84/28**

(84) Benannte Vertragsstaaten: **BE CH FR LI NL**

(56) Entgegenhaltungen:
**EP - A - 0 011 235**
**DE - A - 2 839 033**
**US - A - 4 062 880**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Preuss, Wolfgang, Dr., Finkenweg 12, D-4019 Monheim (DE)**

**EP 0 040 347 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die offengelegte europäische Patentanmeldung 79101036.6 (EP-A-004 913) schildert unter anderem ein Verfahren zur Herstellung von 17-C-Steroid--α-propionsäureverbindungen — insbesondere zur Herstellung von 3-Oxo-pregna-4-en-20-carbonsäure (Δ4-BNC) und/oder 3-Oxo-pregna-1,4-dien-20-carbonsäure (Δ1,4-BNC) — durch mikrobiellen Seitenkettenabbau an 17 C-Seitenketten-steroidsubstraten. Durch Verwendung in bestimmter Weise gezüchteter und ausgewählter Mikroorganismen-Defektmutanten, die auch in Abwesenheit von den Steroidringabbau und/oder das Wachstum hemmenden Inhibitoren Steroidverbindungen mit dem 17 C-α-Propionsäurerest liefern, gelingt die Gewinnung von Δ4-BNC und insbesondere Δ1,4-BNC in technischen Mengen. Eine weitere Ausgestaltung dieses Verfahrens ist in der europäischen Patentanmeldung 79104165.0 (EP-A-0 015 308) beschrieben.

In den älteren europäischen Patentanmeldungen 81100145.2 (EP-A-34 248) und 81100146.0 (EP-A-33 439) wird die Gewinnung von 20-Carboxy-pregna-1,4,17(20)-trien-3-on (Δ1,4,17-BNC) durch mikrobiellen Seitenkettenabbau an 17 C-Seitenketten-Steroidsubstraten beschrieben. Geschildert ist dort weiterhin die Umwandlung der 20-Carbonsäuregruppe in den entsprechenden Ester bzw. das Carbonsäurechlorid.

Die US-PS 3 994 933 beschreibt die Herstellung von 3-Oxo-pregna-4,17(20)-dien-20-carbonsäure (Δ4,17-BNC), ihren niederen Alkylestern und pharmakologisch verträglichen Salzen. Die Säure wird auch hier durch mikrobiellen Seitenkettenabbau von 17 C-Steroidverbindungen gewonnen.

Diese durch Seitenkettenabbau von natürlichen Sterinverbindungen gebildeten BNC-Verbindungen (Δ1,4-BNC, Δ1,4,17-BNC und Δ4,17-BNC) tragen nur in Position 3 eine funktionelle Gruppe im Ringsystem. Alle pharmakologisch wirksamen Corticosteroide enthalten jedoch zusätzliche Sauerstofffunktionen und gegebenenfalls weitere funktionelle Gruppen im Molekül. Besonders wichtig sind dabei die Positionen 9, 11, 17 und 21. Üblicherweise werden einige der Sauerstoffunktionen chemisch eingeführt; dazu gehören insbesondere die Positionen 17 und 21.

Die Oxidation der Position 11 in Steroidverbindungen erfolgt dagegen bevorzugt mikrobiell. In der Fachliteratur ist eine Vielzahl solcher mikrobieller Steroidoxidationen beschrieben. Verwiesen wird in diesem Zusammenhang auf die folgenden Literaturreferate sowie die dort genannten Originalveröffentlichungen:

F. Drawert «Biosynthese von Hydroxy-Verbindungen»; Houben-Weyl «Methoden der organischen Chemie» (1978) 6/1d, Seiten 378 - 388

T. H. Stoudt, Adv. Appl. Microbiol. 2 (1960) Seiten 190 - 195 sowie

Handbuch W. Charney und H. L. Herzog «Microbial Transformations of Steroids» Academic Press (1967) New York, Seite 29.

Bestimmte BNC-Verbindungen mit einer Sauerstoffunktion in 11-Position und insbesondere mit 11-Hydroxyl-gruppen werden in der DE-OS 28 39 033 beschrieben. Geschildert sind hier u.a. die 11α- und die 11β-Hydroxy-Δ1,4-BNC und ihre Herstellung. Die Druckschrift schildert weiterhin die Dehydratisierung dieser Verbindungen zu den entsprechenden, jedoch eine zusätzliche Doppelbindung in 9(11)-Stellung enthaltenden BNC-Verbindungen. Die Dehydratisierung kann in an sich bekannter Weise durchgeführt werden, beispielsweise indem man die 11-Hydroxyl-Verbindungen durch Umsetzung mit Methansulfonsäurechlorid oder p--Toluolsulfonsäurechlorid in die entsprechenden Sulfonsäureester überführt und durch Behandeln mit schwachen Basen wie Natriumacetat in die entsprechenden Δ9,11-Steroide überführt. Die Dehydratisierung kann aber auch in der Weise durchgeführt werden, dass man die genannten Ausgangsverbindungen in einem inerten Lösungsmittel wie Benzol, Toluol oder Xylol mit Mineralsäuren — beispielsweise Schwefelsäure, Phosphorsäure oder Salzsäure — erhitzt.

In 11-Stellung entsprechend hydroxylierte Δ-17(20)-BNC-Verbindungen und ihre Herstellung, insbesondere die 11β-Hydroxy-Δ4,17(20)-BNC und 11β-Hydroxy-1,4,17(20)-BNC sind in der parallelen europäischen Patentanmeldung 81103423.0 (EP-A-39 894) («Neue Δ17(20)-BNC-Verbindungen und Verfahren zu ihrer Herstellung - Priorität Österreich A 2534/80 vom 12.5.1980) der Anmelderin geschildert.

Die nicht vorveröffentlichte europäische Patentanmeldung 79104372.2 (EP-A-11 235) schildert 9-hydroxylierte BNC-Verbindungen und Verfahren zu ihrer Herstellung. Beschrieben ist insbesondere die 9α-Hydroxy-pregna-4,17(20)-dien-3-on-20-carbonsäure.

9α-OH-Δ4-BNC beschreibt die US-PS 4 062 880. Erwähnt ist hier auch die Umwandlung ihres Methylesters zum Ester der Δ4,9(11)-BNC durch Dehydratisierung.

Die vorliegende Erfindung geht von der Aufgabe aus, neue Steroidcarbonsäuren bzw. entsprechende Carbonsäurederivate mit einer Carboxylgruppenfunktion in 20-Stellung zu schaffen, die eine zusätzliche Doppelbindung in 9(11)-Stellung aufweisen. Die Erfindung will damit neue wertvolle Verbindungen der Steroidchemie schaffen, die insbesondere auch als Zwischenprodukte für die Herstellung pharmakologisch aktiver Steroidverbindungen geeignet sind.

Gegenstand der vorliegenden Erfindung sind dementsprechend in einer ersten Ausführungsform neue Δ4,9(11)-sowie wenigstens eine weitere Doppelbindungen in 1(2)- und/oder 17(20)-Stellung aufweisende Pregna-3-on-20-carbonsäureverbindungen der allgemeinen Formel I

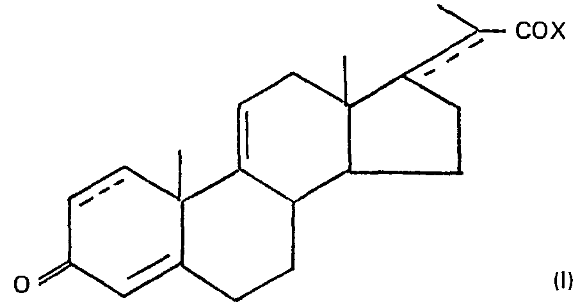

(I)

in der X eine der folgenden Bedeutungen hat: Hydroxyl, OR, wobei R einen Kohlenwasserstoffrest bedeutet, Halogen oder $NH_2$, wobei jedoch solche Verbindungen der allgemeinen Formel I vom Schutz ausgeschlossen sind, in denen X die Bedeutung OH oder OR hat. Die Halogene besonderer Bedeutung sind Brom und insbesondere Chlor.

Erfindungsgemäss werden damit neue BNC-Verbindungen beschrieben, die wenigstens 3-fach olefinisch ungesättigt sind, dabei im A-Ring des Steroidsystems die 4-en-3-on- oder 1,4-dien-3-on-Struktur aufweisen, in 20-Stellung eine Carboxylgruppe oder als funktionelle Derivate eine Estergruppierung, eine Carbonsäurehalogenidgruppe oder eine Carbonsäureamidgruppe aufweisen, zusätzlich eine olefinische Doppelbindung in 9(11)-Stellung besitzen und gegebenenfalls eine weitere olefinische Doppelbindung in 17(20)-Stellung besitzen können, wobei jedoch die durch die Offenbarung der DE-OS 28 39 033 betroffenen Verbindungen hier nicht noch einmal beansprucht werden.

Liegt in 20-Stellung ein Esterrest vor, so weist der veresternde Alkohol vorzugsweise nicht mehr als 20 Kohlenstoffatome auf. Es kann sich dabei um beliebige aliphatische, alicyclische oder aromatische Alkohole handeln. Bevorzugt sind Alkohole mit nicht mehr als 10 C-Atomen. Besondere Bedeutung besitzen die niederen Alkohole mit 1 - 5 C-Atomen. Reste von geradkettigen oder verzweigten Alkanolen können besondere Bedeutung besitzen.

Es hat sich im Rahmen der Efindung weiterhin gezeigt — und dies ist ein weiterer Gegenstand der vorliegenden Erfindung —, dass die neuen 9(11)-ungesättigten Verbindungen in einfacher Weise aus den strukturanalogen Vorstufen hergestellt werden können, die in 9(11)-Stellung gesättigt sind und dabei entweder in 11-Stellung oder in 9-Stellung eine Hydroxylgruppe tragen. Die Hydroxylgruppe in Position 9 liegt in $\alpha$-Stellung vor, in Position 11 kann die 11$\beta$-Stellung bevorzugt sein. Werden Ausgangsverbindungen dieser Art einer Dehydratisierung unterworfen, so kann Wasser in 9(11)-Stellung unter gleichzeitiger Ausbildung der erfindungsgemäss gewünschten olefinischen Bindung in dieser Position abgespalten werden.

Die Ausgangsverbindungen für das erfindungsgemässe Verfahren sind dem zitierten Stand der Technik bzw. den genannten älteren parallelen Anmeldungen der Anmelderin zu entnehmen. Ihre Dehydratisierung zu den erfindungsgemässen Verbindungen der Formel I erfolgt vorzugsweise unter Anwendung von Dehydratisierungsmitteln bei nicht oder nur mässig erhöhten Temperaturen. Bevorzugt sind insbesondere Temperaturen bis maximal etwa 80°C, insbesondere kann es wünschenswert sein, Temperaturen von etwa 50°C nicht zu überschreiten. Das Arbeiten im Temperaturbereich von —10°C bis +25°C kann besonders zweckmässig sein.

Alle bekannten chemischen Dehydratisierungsmittel zur Wasserabspaltung aus sekundären oder tertiären Alkoholen sind zur Durchführung des erfindungsgemässen Verfahrens geeignet, sofern sie nicht unerwünschte Nebenreaktionen mit dem Ausgangsmaterial eingehen. Saure Dehydratisierungsmittel insbesondere entsprechende Mineralsäuren — z.B. Schwefelsäure, Phosphorsäure oder Salzsäure — bzw. Mineralsäurederivate können bevorzugt sein. Möglich ist auch eine Behandlung mit N-Halogenamiden bzw. N-Halogenimiden und $SO_2$, wie in der GB-PS 869 815 beschrieben. Die zu dehydratisierenden Verbindungen können dabei in inerten Lösungsmitteln, beispielsweise Kohlenwasserstoffverbindungen, gelöst sein.

Ein besonders wichtiger Aspekt der Erfindung betrifft Verbindungen der allgemeinen Formel I und ihre Herstellung, wobei X in dieser allgemeinen Formel Halogen und insbesondere Chlor bedeutet. Diese Säurehalogenide sind beispielsweise wichtige Zwischenprodukte für nachfolgende chemische Reaktionen unter Umwandlung des Substituenten in 17-Stellung des Steroidgerüstes. Überraschenderweise gelingt die Bildung der erfindungsgemäss gewünschten 9(11)-en-20-carbonsäurehalogenide bereits unter aussergewöhnlich milden Reaktionsbedingungen, bei denen die unerwünschte Mitreaktion anderer reaktiver Stellen der zugrunde liegenden mehrfach ungesättigten BNC-Struktur noch nicht stattfindet.

In einer bevorzugten Ausführungsform des erfindungsgemässen Verfahrens kann die Dehydratisierung zur 9(11)-en-Bindung mit der Bildung des 20-Carbonsäurehalogenids verbunden werden. Hier hat sich gezeigt, dass die Anwendung üblicher Halogenierungsmittel insbesondere die Anwendung von Thionylhalogenid im Überschuss zusammen mit einer tertiären N-Base unmittelbar zur Bildung des 9(11)-ungesättigten 20-Carbonsäurehalogenids aus den entsprechenden 9- bzw. 11-Hydroxy-20-carbonsäuren führen kann. Das gebildete Carbonsäurehalogenid kann dann leicht entweder zur freien Säure hydrolysiert werden, mit Alkoholen der angegebenen Art zu entsprechenden Estern umgesetzt werden, oder mit Ammoniak oder einer Ammoniak liefernden Verbindung zum Carbonsäureamid umgewandelt werden. Auf die hier geschilderte Weise werden die verschiedenen neuen Stoffe der Erfindung bequem zugänglich.

Wie bereits erwähnt, gelingt überraschenderweise die Bildung der erfindungsgemäss gewünschten 9(11)-en-20-Carbonsäurehalogenide unter derart milden Reaktionsbedingungen, dass die unerwünschte Mitreaktion anderer reaktiver Stellen der zugrunde liegenden mehrfach ungesättigten BNC-Struktur noch nicht stattfindet. So zeigt sich, dass beispielsweise eine praktisch quantitative Dehydratisierung und gleichzeitige Säurechloridbildung stattfindet, wenn die folgenden Reaktionsbedingungen eingehalten werden: Reaktionstemperaturen unter 15°C, Arbeiten in Gegenwart eines inerten Verdünnungsmittels und in Anwesenheit einer basischen Verbindung. Als inerte Lösungsmittel kommen beispielsweise halogenierte Kohlenwasserstoffe oder — mit Einschränkungen — Ether in Betracht. Geeignete inerte Lösungsmittel sind beispielsweise Methylenchlorid oder Chloroform. Als basische Verbindung sind vor allem tertiäre N-Basen, insbesondere Pyridin oder Dimethylformamid zu nennen, die zweckmässig in Mengen von wenigstens 2 Mol Base, vorzugsweise von wenigstens 3 Mol Base pro Mol Steroidcarbonsäure zur Verwendung kommen.

Das Halogenierungsmittel wird in der zuletzt geschilderten Ausführungsform der gleichzeitigen Dehydratisierung und Ausbildung der Carbonsäurehalogenidgruppierung im Überschuss über die zur Carbonsäurehalogenidbildung erforderlichen Menge eingesetzt. In der Regel wird das Halogenierungsmittel wenigstens in etwa 1-molarem Überschuss verwendet, wobei es weiterhin bevorzugt sein kann, einen zu grossen Überschuss zu vermeiden. Bevorzugt liegt der Überschuss des Halogenierungsmittels im Bereich von 1- bis 3-molar, vorzugsweise von 1- bis 1,5-molar. Als Halogenierungsmittel kommen Phosphorhalogenide, insbesondere $PCl_3$ oder $PCl_5$ bzw. die entsprechenden Bromide, vor allen Dingen jedoch das Thionylhalogenid und hier insbesondere das Thionylchlorid in Betracht. Das Halogenierungsmittel wird zweckmässigerweise zu der Lösung der umzusetzenden Steroidverbindung im inerten Lösungsmittel gegeben. Es hat sich als vorteilhaft erwiesen, das Halogenierungsmittel in möglichst reiner Form einzusetzen. Offenbar fördern im Halogenierungsmittel vorliegende Verunreinigungen unerwünschte Nebenreaktionen. Zweckmässig ist beispielsweise eine Reinigung des Halogenierungsmittels mit einer ungesättigten Verbindung wie Leinöl oder besonders Squalen. Diese ungesättigten Komponenten reagieren mit den Verunreinigungen im Halogenierungsmittel und senken damit die Bildung unerwünschter Nebenprodukte auf ein Minimum.

Liegen als Ausgangsmaterialien für die Bildung der Carbonsäurehalogenide bereits 9(11)-ungesättigte Carbonsäuren im Sinne der allgemeinen Formel I vor, dann kann die Bildung der entsprechenden Säurehalogenide, insbesondere des entsprechenden Säurechlorids, in gleicher Weise wie zuvor beschrieben, vorgenommen werden. Hierbei kann es allerdings bevorzugt sein, mit nur einem sehr begrenzten Überschuss des Halogenierungsmittels zu arbeiten, der vorzugsweise nicht über 20 Mol-% und insbesondere nicht über 10 Mol-% beträgt. Besonders geeignete Reaktionsbedingungen für eine solche Reaktion sind: Reaktionstemperaturen unter 10°C, vorzugsweise unter 5°C, stöchiometrische Mengen der Reaktanten oder nur sehr begrenzter Überschuss des Halogenierungsmittels (insbesondere Thionylchlorid), der die zuvor angegebenen Grenzen zweckmässig nicht überschreitet, sowie kurze Reaktionszeiten, die vorzugsweise 30 Minuten nicht überschreiten. Besonders geeignet kann die folgende Kombination von Umsetzungsbedingungen sein: 0°C, Thionylchloridüberschuss von 0 - 10 Mol-% und Reaktionsdauer von 15 - 20 Minuten in Gegenwart eines inerten Lösungsmittels. Es ist in dem hier geschilderten Fall kein Zusatz eines Katalysators — beispielsweise Pyridin oder Dimethylformamid — und kein Zusatz einer grösseren Menge an Base wie beispielsweise tertiäres Amin oder Alkalicarbonat erforderlich.

Aus den Säurehalogeniden können die erfindungsgemässen neuen Carbonsäureamide der allgemeinen Formel I gewonnen werden, indem man das 20-Carbonsäurehalogenid mit Ammoniak oder einer Ammoniak liefernden Verbindung zur Umsetzung bringt. Diese Umsetzung wird zweckmässigerweise im Temperaturbereich von etwa —20°C bis etwa 80°C, vorzugsweise im Bereich von etwa —5°C bis etwa 35°C durchgeführt. Ammoniak bzw. die unter den Reaktionsbedingungen Ammoniak liefernde Verbindung wird wenigstens in etwa äquimolaren Verhältnissen eingesetzt. Geeignet sind beispielsweise Mengen des Ammoniaks bzw. der Ammoniak liefernden Verbindung von 1,1 bis 5 Äquivalenten — bezogen auf Säurehalogenid — vorzugsweise Mengen von etwa 1,2 bis etwa 3 Äquivalenten. Wird nicht Ammoniak selber, sondern eine Ammoniak liefernde Verbindung eingesetzt, so eignet sich hierfür insbesondere Ammoniumhydroxyd.

Die Umsetzung des 20-Carbonsäurehalogenids mit Ammoniak bzw. der Ammoniak liefernden Verbindung erfolgt vorzugsweise ebenfalls in einem organischen Lösungsmittel, beispielsweise in Halogenkohlenwasserstoffen, wie zuvor angegeben. Ein besonders geeignetes inertes Lösungsmittel ist auch hier Methylenchlorid oder Chloroform.

Wird Ammoniumhydroxyd als Ammoniak liefernde Komponente eingesetzt, so fällt im Reaktionsgemisch neben der organischen Phase eine wässrige Phase an. Durch einfache Phasentrennung oder auch durch Abtrennung des fest ausgefallenen Amids gelingt die Gewinnung des Reaktionsproduktes. Die abgetrennte organische Phase wird zweckmässigerweise mehrfach mit Wasser gewaschen, anschliessend mit beispielsweise Calciumsulfat getrocknet und gefiltert. Das als inertes Verdünnungsmittel eingesetzte organische Lösungsmittel wird abgetrennt, die Carboxamidoverbindung kann dann in an sich bekannter Weise weiter gereinigt werden.

Bei der Umsetzung zwischen Carbonsäurehalogenid und Ammoniak freiwerdende Halogenwasserstoffsäure wird entweder durch einen Überschuss von Ammoniak bzw. Ammoniumhydroxyd gebunden, es kann aber auch eine basische Komponente zur Bindung der freiwerdenden Säure mitverwendet werden.

Die neuen Verbindungen der allgemeinen Formel I sind wertvolle Produkte der Steroidchemie und insbesondere wichtige Zwischenprodukte bei der Herstellung pharmakologisch aktiver Corticosteroide. Sie ermöglichen beispielsweise die Einführung von Substituenten in 9- und/oder 11-Stellung, die zu einer Modifikation der pharmakologischen Aktivität führen können.

*Beispiel 1*

*Pregna-1,4,9(11)-trien-3-on-20-carbonylchlorid*

718 mg 11β-Hydroxy-pregna-1,4-dien-3-on-20-carbonsäure in 20 ml trockenem Methylenchlorid werden bei 0°C mit 0,3 ml trockenem Pyridin und 0,6 ml frisch über Squalen destilliertem Thionylchlorid versetzt. Nach 1 h bei 0°C wird im Vakuum zur Trockne eingeengt. Den noch schmierigen Rückstand löst man wieder in wenig Methylenchlorid und engt noch einmal zur Trockne ein.

Das IR-Spektrum des Rückstands in $CHCl_3$ zeigt, dass die Umsetzung zum Carbonsäurechlorid vollständig ist (Banden bei 1777, 1665, 1638 (Schulter), 1628, 1609 $cm^{-1}$).

Das so erhaltene rohe Pregna-1,4,9(11)-trien-3-on-20-carbonylchlorid kann ohne Reinigung weiter

umgesetzt werden. Die Ausbeutebestimmung erfolgt am besten nach Veresterung des Säurechlorids.

*Beispiel 2*

*Bestimmung der Ausbeute an Pregna-1,4,9(11)-trien-3-on-20-carbonylchlorid aus Beispiel 1 durch Überführung in den Methylester (Pregna-1,4,9(11)-trien-3-on-20-carbonsäuremethylester)*

Das nach Beispiel 1 aus 718 mg der Säure hergestellte rohe Säurechlorid wird in 15 ml trockenem Methylenchlorid gelöst und mit 0,6 ml Pyridin sowie 2 ml Methanol versetzt. Nach 1 h wird mit 20 ml $CH_2Cl_2$ verdünnt und die organische Phase nacheinander mit Wasser, verdünnter $H_2SO_4$ und wieder mit Wasser gewaschen, dann getrocknet und schliesslich eingeengt. Als Rückstand verbleiben 700 mg des rohen dreifach ungesättigten Methylesters.

Das $^1$H-NMR-Spektrum bestätigt die postulierte Struktur. Durch quantitative Dünnschichtchromatographie ergibt sich eine Ausbeute von 86%, bezogen auf die eingesetzte Säure.

$^1$H-NMR (80 MHz, $CDCl_3$, $\delta$-Werte):
0,70 (18-$CH_3$, s); 1,17 (21-$CH_3$, dJ = 6,9 Hz), 1,40 (19-$CH_3$, s); 3,64 (-$OCH_3$, s); 5,48 (11-CH);
6,05;

| | | |
|---|---|---|
| 6,18; | 6,20; | ABC-System von |
| 6,31; | 6,33; | 1-CH, 2-CH, 3-CH |
| 7,11; | 7,23; | |

*Beispiel 3*

*Pregna-1,4,9(11)-trien-3-on-20-carboxamid*

Nach Beispiel 1 aus 718 mg 11$\beta$-Hydroxy-pregna-1,4-dien-3-on-20-carbonsäure hergestelltes Pregna-1,4,9(11)-trien-3-on-20-carbonylchlorid wird in 20 ml trockenem $CH_2Cl_2$ gelöst. Bei 0°C leitet man über 2 h langsam gasförmiges $NH_3$ durch die Lösung, giesst dann in die gleiche Menge Eiswasser und säuert mit verdünnter Salzsäure vorsichtig an.

Die Phasen werden getrennt, man wäscht die wässrige Phase 2 × mit Methylenchlorid nach und trocknet die vereinigten $CH_2Cl_2$-Phasen über $Na_2SO_4$. Nach Abtrennen des Trockenmittels und Einengen des Lösungsmittels verbleiben 650 mg Feststoff, aus dem durch Chromatographie über Kieselgel [Laufmittel: $CH_2Cl_2$ (85), Essigester (10) und Ethanol (5)] 500 mg des gewünschten Amids erhalten werden.

$^1$H-NMR (80 MHz, $CDCl_3$, $\delta$-Werte):
0,71 (18-$CH_3$, s); 1,20 (21-$CH_3$, d, J = 6,4 Hz); 1,40 (19-$CH_3$); 5,48 (11-CH);
6,05;

| | | |
|---|---|---|
| 6,18; | 6,20; | ABC-System von |
| 6,31; | 6,33; | 1-CH, 2-CH, 3-CH |
| 7,12; | 7,25; | |

**Patentansprüche**

1. $\Delta$4,9(11)-sowie wenigstens eine weitere Doppelbindung in 1(2)- und/oder 17(20)-Stellung aufweisende Pregna-3-on-20-carbonsäureverbindungen der allgemeinen Formel I

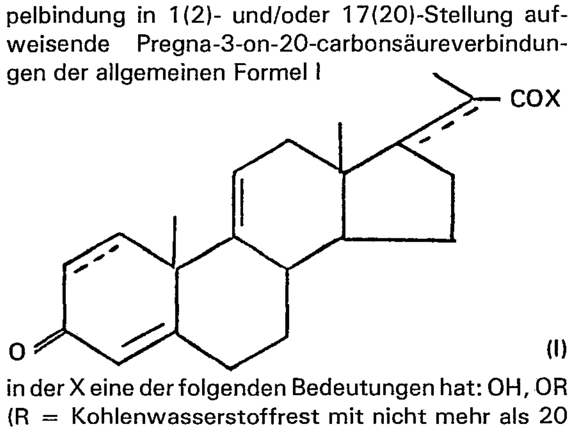

(I)

in der X eine der folgenden Bedeutungen hat: OH, OR (R = Kohlenwasserstoffrest mit nicht mehr als 20 C-Atomen), Halogen — insbesondere Chlor oder Brom — oder $NH_2$, wobei solche $\Delta$1,4,9(11)-Verbindungen der allgemeinen Formel I vom Schutz ausgenommen sind, in denen X die Bedeutung von OH oder OR hat.

2. Verfahren zur Herstellung von Verbindungen der allgemeinen Formel I, dadurch gekennzeichnet, dass man die Struktur-analogen, jedoch 9 (11)-gesättigten und dabei in 9- oder 11-Stellung hydroxylierten Ausgangsverbindungen in an sich bekannter Weise unter Ausbildung der 9(11)-en-Bindung dehydratisiert und gewünschtenfalls das angefallene Produkt der chemischen Transformation zu den Endprodukten der allgemeinen Formel I unterwirft.

3. Verfahren nach Anspruch 2, dadurch gekennzeichnet, dass man zur Dehydratisierung von den 20-Carbonsäuren beziehungsweise ihren Estern ausgeht.

4. Verfahren nach Anspruch 2, dadurch gekennzeichnet, dass man von 9$\alpha$- oder insbesondere 11$\beta$-hydroxylierten Ausgangsverbindungen ausgeht.

5. Verfahren nach Anspruch 2, dadurch gekennzeichnet, dass man zur Herstellung von Verbindungen der allgemeinen Formel I, in der X Halogen oder $NH_2$ bedeutet, die zugehörige Steroid-20-Carbonsäure mit einem Halogenierungsmittel bei Temperaturen nicht über 15°C, vorzugsweise nicht über 5°C umsetzt und gewünschtenfalls das gebildete Steroid-20-Carbonsäurehalogenid mit Ammoniak oder einer Ammoniak liefernden Verbindung umsetzt.

6. Verfahren nach Anspruch 5, dadurch gekennzeichnet, dass man als Halogenierungsmittel Thionylhalogenid, insbesondere Thionylchlorid einsetzt.

7. Verfahren nach Ansprüchen 2 bis 6 dadurch gekennzeichnet, dass man die 9(11)-Dehydratisierung und die Bildung des Steroid-20-Carbonsäurehalogenids in einem Verfahrensschritt durchführt, in dem man das Halogenierungsmittel im Überschuss über die zur Bildung des Carbonsäurehalogenids benötigte Menge einsetzt, wobei zweckmässig mit etwa 1- bis 1,5-molarem Überschuss gearbeitet wird.

**Claims**

1. $\Delta$4,9(11)-pregna-3-one-20-carboxylic acid compounds containing at least one other double bond in the 1(2)- and/or 17(20)-position and corresponding to the following general formula

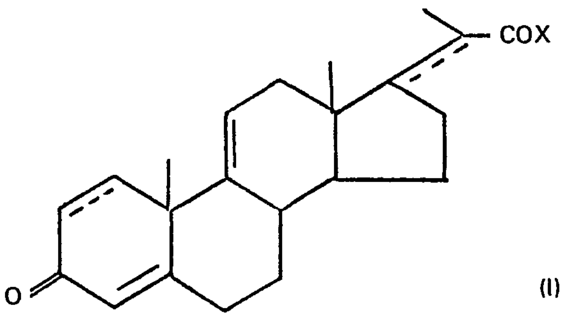

(I)

in which X has one of the following meanings: OH, OR (R = a hydrocarbon radical containing no more than 20 carbon atoms), halogen, particularly chlorine or bromine, or $NH_2$, those Δ1,4,9(11)-compounds of general formula I in which X represents OH or OR being excluded from protection.

2. A process for producing compounds corresponding to general formula I, characterized in that the structurally analogous, but 9(11)-saturated starting compounds hydroxylated in the 9- or 11--position are dehydrated in known manner to form the 9(11)-ene bond and, if desired, the product obtained is subjected to chemical transformation to form the end products corresponding to general formula I.

3. A process as claimed in claim 2, characterized in the 20-carboxylic acids or their esters are used as starting materials for the dehydration reaction.

4. A process as claimed in claim 2, characterized in that 9α-hydroxylated or, more particularly, 11β--hydroxylated starting compounds are used.

5. A process as claimed in claim 2, characterized in that, for producing compounds of general formula I, in which X is halogen or $NH_2$, the associated steroid-20-carboxylic acid is reacted with a halogenating agent at temperatures not exceeding 15°C and preferably at temperatures not exceeding 5°C and, if desired, the steroid-20-carboxylic acid halide formed is reacted with ammonia or with an ammonia-yielding compound.

6. A process as claimed in claim 5, characterized in that thionyl halide, more particularly thionyl chloride, is used as the halogenating agent.

7. A process as claimed in claims 2 to 6, characterized in that the 9(11)-dehydration reaction and formation of the steroid-20-carboxylic acid halide are carried out in a single step by using the halogenating agent in an excess over and above the quantity required for forming the carboxylic acid halide, an approximately 1 to 1.5-molar excess best being used.

**Revendications**

1. Dérivés d'acides Δ4,9(11)-prégna-3-one-20--carboxyliques et présentant au moins une autre double liaison en position 1(2) et/ou en position 17(20), de formule générale I

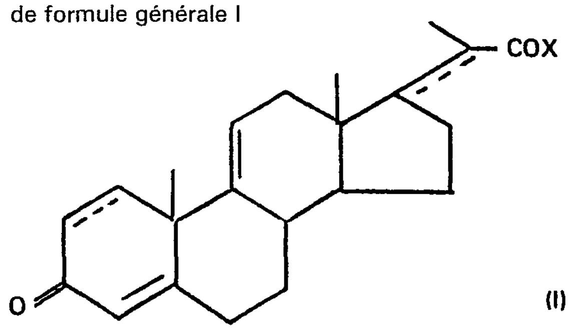

(I)

dans laquelle X a l'une des significations suivantes: OH, OR (R = reste d'hydrocarbure n'ayant pas plus de 20 atomes de carbone), halogène — en particulier chlore ou brome — ou $NH_2$, les composés Δ1,4,9-(11) de formule générale I dans lesquels X représente OH ou OR étant exclus de la protection.

2. Procédé pour la fabrication de composés de formule générale I, caractérisé en ce que l'on déshydrate de manière connue les composés de départ de structure analogue, mais saturés en 9(11) et dans ce cas hydroxylés en position 9 ou en position 11, avec formation de la liaison 9(11)-ène et, si on le désire, on soumet le produit formé à la transformation chimique en les produits de formule générale I.

3. Procédé selon la revendication 2, caractérisé en ce que, pour la déshydratation, on part des acides 20-carboxyliques ou de leurs esters.

4. Procédé selon la revendication 2, caractérisé en ce que l'on part de composés de départ 9α- ou en particulier 11β-hydroxylés.

5. Procédé selon la revendication 2, caractérisé en ce que, pour la fabrication de composés de formule générale I, dans laquelle X représente un halogène ou $NH_2$, on fait réagir l'acide stéroïde-20-carboxylique correspondant avec un agent halogénant à des températures de pas plus de 15°C, de préférence pas plus de 5°C, et, si on le désire, on fait réagir l'halogénure d'acide stéroïde-20-carboxylique formé avec l'ammoniac ou un composé libérant de l'ammoniac.

6. Procédé selon la revendication 5, caractérisé en ce que l'on utilise comme agent halogénant un halogénure de thionyle, en particulier le chlorure de thionyle.

7. Procédé selon les revendications 2 à 6, caractérisé en ce que l'on effectue la 9(11)-déshydratation et la formation de l'halogénure d'acide stéroïde-20--carboxylique en une étape opératoire, en utilisant l'agent halogénant en excès par rapport à la quantité nécessaire pour la formation de l'halogénure d'acide carboxylique, en opérant avantageusement avec un excès environ 1- à 1,5-molaire.